(19) Europäisches Patentamt
European Patent Office
Office européen des brevets

(11) Publication number: **0 158 996 B1**

# (12) EUROPEAN PATENT SPECIFICATION

(45) Date of publication of patent specification: **04.12.91**

(51) Int. Cl.[5]: **A61K 31/025**, A61K 9/00

(21) Application number: **85104491.7**

(22) Date of filing: **12.04.85**

## (54) Perfluoro carbon compound aqueous dispersions.

(30) Priority: **16.04.84 US 600653**

(43) Date of publication of application:
**23.10.85 Bulletin 85/43**

(45) Publication of the grant of the patent:
**04.12.91 Bulletin 91/49**

(84) Designated Contracting States:
**AT BE CH DE FR GB IT LI LU NL SE**

(56) References cited:
**EP-A- 0 072 234**
**EP-A- 0 105 584**
**US-A- 3 958 014**
**US-A- 4 105 798**

(73) Proprietor: **AFFINITY BIOTECH, INC.**
**305 Chelsea Parkway Chelsea Business Park**
**Boothwyn, Pennsylvania 19061(US)**

(72) Inventor: **McCormick, William**
**284 Acton Street**
**Carlisle Massachusetts(US)**

(74) Representative: **Madgwick, Paul Roland et al**
**Ladas & Parry Isartorplatz 5**
**W-8000 München 2(DE)**

**EP 0 158 996 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

This invention relates to aqueous dispersions of perfluoro compounds useful as gas transfer agents and for drug delivery and other therapeutic applications in animals including man. Typical perfluoro compounds, dispersions and uses are described in U.S. Patents 3,911,138 to Clark and 4,105,798 to Moore and Clark. The perfluoro compounds described in these patents are essentially non-toxic and therefore eminently suitable for therapeutic uses.

Aqueous dispersions of perfluoro compounds, such as those of the U.S. patents cited above, are prepared by dissolving a surfactant in water, adding the perfluoro compound, and then agitating the mixture until a uniform dispersion of the perfluoro compound is obtained. Since the perfluoro compounds are extremely hydrophobic, considerable mechanical energy is required for effective dispersion, such as high pressure homogenization or sonically induced cavitation. Depending on selection of perfluoro compound, surfactant and proportions of ingredients, highly stable dispersions of very small average particle size, on the order of about 0.1 to 1.0 $\mu$m (micron) in diameter, are obtained. In such cases, the dispersions are essentially transparent and are sometimes described in the literature as "microemulsions" (U.S. Patent No. 3,778,381 to Rosano et al). However, in other cases useful dispersions can be prepared having larger average particle sizes and these may be borderline between suspensions and emulsions. Accordingly, the term "dispersion" is used in this specification to indicate, generically, any two-phase system, whether a suspension, emulsion or microemulsion, and whether oil-in-water, water-in-oil or invert, and the term "perfluoro compound phase" means the perfluoro compound-containing particles of the dispersion. For purposes of illustration, the emphasis in this specification will be on the more preferred compositions, viz., emulsions or microemulsions of the oil-in-water type.

To overcome the difficulty of forming good dispersions with the extremely hydrophobic perfluoro compounds it is common practice to prepare the dispersions using a high proportion of surfactant to perfluoro compound (of the order of about 1:5 by weight) and low concentrations of perfluoro compound (about 20-25% w/v). This facilitates not only good dispersability but also small particle size. The resulting dispersions have good stability and low viscosity which promote their rapid transit throughout the cardiovascular system, particularly in capillaries or vessels which are blocked or constricted. Such dispersions will also exhibit reduced retention in the reticuloendothelial system (RES). However, the goal of optimizing the dispersion process and physical qualities of the dispersion (particularly stability and viscosity) imposes a practical upper limit on the amount of perfluoro compound in the finished dispersions and thereby also limits the gas transfer capacity of the dispersions and their capacity for treatment of hypoxic cells and for carrying lipophilic drugs in the manner described in European Patent Publication A 105584.

Additionally, while it is possible (although not always practical because of formulation difficulties) to select surfactants and/or dosages thereof which are sufficiently non-toxic by $LD_{50}$ standards, other toxic responses attributable to the surfactants, such as complement activation, have been reported. This has led either to excluding the surfactants from use in perfluoro compound dispersions or to reduction in their concentration with proportionate reduction in the amount of perfluoro compound which can be effectively dispersed.

In summary, it has heretofore been considered necessary, in order to satisfy the requirements of efficient dispersability, low particle size, stability and viscosity on the one hand, and sufficient gas transfer capacity and biological compatibility on the other, to maintain fairly low concentrations of perfluoro compound in aqueous dispersions thereof, on the order of no more than about 25% (w/v), i.e., about 25g/100ml. of the total dispersion, so that the amount of surfactant may also be kept low, on the order of no more than about 2-5% (w/v), i.e., about 2-5g/100ml. of the total dispersion.

It has now been found, in accordance with the invention, that the amount of surfactant heretofore regarded as necessary in forming high quality, biologically compatible, therapeutic aqueous dispersions of perfluoro compounds, can be substantially and even drastically reduced without diminishing ability to maintain the perfluoro compounds in stable, uniform dispersion and without sacrificing those properties of the dispersions which contribute to therapeutic efficacy, such as low viscosity, stability and high amounts of perfluoro compound. Quite the contrary, the perfluoro compound dispersions can now be even more efficiently prepared and can contain higher amounts of the perfluoro compound than heretofore deemed possible, but with the low particle size, low viscosity and good stability which are the hallmarks of the earlier dispersions described in the Clark and Moore patents cited above.

These seemingly contradictory, but highly beneficial results are obtained (in one method of practice of the invention) by (a) providing an initial aqueous dispersion of perfluoro compound and surfactant, (b) concentrating the perfluoro compound phase of the initial dispersion, (c) separating all or part of the concentrated perfluoro compound phase from the aqueous phase, and (d) redispersing the concentrated

perfluoro compound phase in an aqueous medium. "Concentrating" as the term is used herein, means condensing, amassing or gathering together the perfluoro compound particles to form a perfluoro compound phase more concentrated in perfluoro compound than in the initial dispersion. The redispersion may be achieved with sterile water alone or redispersion may be facilitated by addition of an aqueous medium containing a suitable auxiliary surfactant or other agent. Moreover, as hereinafter described, the foregoing concentration, separation and redispersion steps may be practiced in a continuous manner and/or may be combined with classification of the concentrated perfluoro compound phase particles with consequent even greater uniformity of particle size.

In one aspect of the invention, therefore, there is provided a process for preparing an aqueous dispersion of a perfluorocompound, which comprises:

(a) providing an initial dispersion comprising (i) a perfluoro compound phase containing surfactant complexed with the perfluoro compound, and (ii) an aqueous phase containing free principal surfactant;
(b) concentrating the perfluoro compound phase;
(c) separating all or a portion of the aqueous phase from the concentrated perfluoro compound phase;
(d) redispersing the concentrated perfluoro compound phase with an amount of an aqueous medium effective to redisperse the perfluoro compound phase and thereby to form a final dispersion having the advantage of reduction, in the final dispersion, of the amount of surfactant heretofore considered necessary for such dispersions.

In another aspect of the invention, the aforesaid method additionally affords the opportunity to use, in preparing the initial dispersion, the higher proportions of surfactant to perfluoro compound known to facilitate more efficient dispersion and the formation of high quality dispersions of perfluoro compounds but without, ultimately, increasing the viscosity to undesirable levels or introducing the unacceptable toxicity associated with many surfactants. For internal use the dispersions of the invention will have viscosities at body temperature (37° C.) of no greater than the viscosity of blood and preferably considerably lower, e.g., from about the viscosity of water (about 0.7 mPas-centipoise) to about 3.0 mPas (centipoise).

In still another aspect of the invention, gas and drug transporting compositions are provided comprising a stable, uniform, aqueous dispersion of a perfluoro compound and at least one surfactant, wherein at least a major proportion of the principal surfactant present is complexed with the perfluoro compound which compositions contain higher amounts of perfluoro compound and lower amounts of surfactant than have heretofore been deemed capable of providing satisfactory dispersions, with proportionately improved capacity for gas transfer and therapeutic effect, and proportionately diminished toxicity attributable to the surfactant.

In a further, highly significant and preferred aspect of the invention, aqueous dispersions of perfluoro compounds are provided which not only have the characteristics described in the foregoing aspects, but also have controlled, small particle size range, low viscosity and good stability. These improvements lead to desirable biological characteristics such as good cardiovascular residence time and low RES retention, and ultimately to greater therapeutic benefit of the nature described in the aforementioned Clark and Moore patents and European Patent Publication-A-105584.

The extraordinary benefits of the invention are based in part upon the discovery that when the perfluoro compound phase of the initial dispersion is concentrated, a portion, often a minor portion, of the surfactant will remain with the perfluoro compound particles and the balance of the surfactant, often the major portion, will be in the aqueous phase. The concentrated perfluoro compound phase is then separated from the aqueous phase and readily redisperses in an aqueous medium which does not contain the surfactant removed with the aqueous phase in the separation step. In some cases the concentration step results in two distinct layers comprising a perfluoro compound phase and an aqueous phase. When practiced continuously, as described below, layering of the phases usually will not be observed due to the dynamic characteristics of the process but concentrating, separation and redispersion nevertheless occur.

It is not fully understood how or why this phenomenon occurs. In some manner a portion of the surfactant binds with or is adsorbed to the perfluoro compound, thus forming perfluoro compound-containing particles during the initial dispersion process and/or the concentrating step in which surfactant is so tightly bound that it remains "complexed" with the perfluoro compound particles during the subsequent concentrating and/or phase separation steps. The other portion of the surfactant remains free (uncomplexed) in the continuous, aqueous phase of the initial dispersion. Thus, an important feature of the invention is that a substantial portion of the surfactant present in the initial dispersion remains as free, uncomplexed surfactant and therefore is separated from the perfluoro compound phase. Accordingly, the free surfactant is not carried through to the final dispersion. The balance of the surfactant remains associated with the perfluoro compound-containing particles in sufficient amounts to permit the redispersion.

Consequently, according to the invention, the initially larger amounts of surfactant (and higher proportion of surfactant to perfluoro compound) known to facilitate initial dispersion can now be used but without their ultimate adverse effects, namely, unacceptable viscosity and/or toxicity of the final dispersion due to such larger amounts of surfactant.

Significantly, also, the larger amounts of surfactant now permissible in the initial dispersion also permit larger amounts of perfluoro compound, e.g., greater than the 20-25% (w/v) heretofore considered the practical upper limit, and therefore provide higher gas transfer capacity than is possible with dispersions containing smaller amounts of perfluoro compound, while permitting suitably small particle size and low viscosity.

For the purposes of this specification, the surfactant remaining with the perfluoro compound particles during the concentration and separation steps is identified as "associated" surfactant, and the surfactant remaining in the separated aqueous liquid is called "free" surfactant. Surfactant employed in forming the initial dispersion (prior to concentrating and separating) is sometimes called "principal" surfactant herein to distinguish it from auxiliary surfactant which optionally may be used for the redispersion step. Where "surfactant" is referred to without qualification, surfactant employed in forming the initial dispersion is understood. The terms "initial dispersion" and "final dispersion" are used herein to mean, respectively, the dispersion provided in the first step of the process of the invention and the dispersion resulting from the subsequent steps of the process.

A major proportion of the associated surfactant in the concentrated perfluoro compound phase is "complexed" with the perfluoro compound, as determined by concentrating the perfluoro compound phase of a sample of a dispersion, measuring the amount of the surfactant in the resulting supernatant layer, and subtracting this amount from the known total amount of surfactant in the sample, thus giving the amount of surfactant "complexed" within the particles of the concentrated perfluoro compound phase. The process of the invention has been practiced successfully and the sample is a dispersion of the invention if the calculated difference is more than the amount of surfactant in the supernatant layer. Often, this indicates that most of the surfactant was removed during the phase concentration and separation steps practiced on the initial dispersion when forming the final dispersion. In any event, it indicates that a major proportion of the associated surfactant in the concentrated perfluoro compound phase is "complexed" with the perfluoro compound in the final dispersion and only a minor, even minute, amount is found in the aqueous phase. Preferably, at least 75% by weight of the surfactant in the final dispersion will be found to be complexed with the perfluoro compound. "Complexed" surfactant, as the term is used herein, denotes surfactant which remains with the concentrated perfluoro compound phase material after removal of the aqueous phase. While it is believed that such surfactant does not chemically combine with the perfluoro compound phase material, the invention is not to be limited by any theory as to the nature of the association, whether physical, chemical or a combination thereof.

In determining the amount of complexed surfactant in an aqueous dispersion of a perfluoro compound, centrifugation conditions are used which result in separation of the dispersion into a supernatant aqueous layer and a lower layer which is still a perfluorocarbon dispersion, the integrity of the dispersion having been retained, but which is more concentrated with respect to perfluoro compound as a result of the removal of water therefrom into the aqueous layer. Preferably, when the dispersion of the invention is centrifuged under the conditions set forth in Runs 4 through 9 in Table I below, a major proportion of the surfactant remains complexed in the perfluoro compound phase. Also, preferably, in some embodiments when the dispersion is centrifuged under the stronger conditions set forth in Run 10 in Table I, a major proportion of the surfactant remains complexed in the perfluoro compound phase.

The perfluoro compounds used in preparing the dispersions of the invention are any fluorinated hydrocarbons or hetero atom containing derivatives thereof which exhibit gas transfer properties, and which are capable of dispersion in an aqueous medium and of systemic administration as aqueous dispersions to animals including man. The compounds may be substantially fluorinated or completely fluorinated and are generally, but not necessarily, liquids at ambient temperature and pressure.

"Substantially fluorinated" in this specification means that most of the hydrogen atoms of a compound have been replaced by fluorine atoms, such that further replacement does not substantially increase the gas transport capability of the material. It is believed that this level is reached when at least about 80-90% of the hydrogen atoms have been replaced by fluorine atoms. However, it is preferred that at least 95% of the hydrogen atoms have been replaced, more preferably at least 98% and even more preferably, 100%. In the aforementioned U.S. patents 3,911,138 and 4,105,798, the ability to transport oxygen is related to the solubility in the materials of a gas such as oxygen. These patents suggest that the perfluorinated materials will absorb 10-100 cc of oxygen per 100 cc of material at 25° C. and 760 milliliters of mercury.

Among the fluorinated materials suitable for use in this invention include those which are broadly described as cyclic perfluorohydrocarbons or derivatives thereof.

Examples are the perfluorinated derivatives of chemically inert $C_9$-$C_{18}$ polycyclic compounds such as bicyclononanes (e.g., bicyclo [3.3.1] nonane, 2,6-dimethylbicyclo [3.3.1] nonane, 3-methylbicyclo [3.3.1] nonane and trimethylbicyclo [3.3.1] nonane); adamantane and alkyl ($C_1$-$C_6$) adamantanes such as methyl and dimethyladamantane, ethyl and diethyladamantane, trimethyladamantane, ethylmethyladamantane, ethyldimethyladamantane and triethyladamantane; methyldiadamantane and trimethyldiadamantane; methyl and dimethylbicyclooctanes; tetrahydrobinor-S, pinane, camphane, decalin and alkyl decalins such as 1-methyldecalin; and 1,4,6,9-dimethanodecalin; bicyclo [4.3.2] undecane, bicyclo [5.3.0] decane, bicyclo [2.2.1] octane, tricyclo [5.2.1.$0^{2,6}$] decane, methyltricyclo [5.2.1.$0^{2,6}$] decane, and the like; or any mixtures thereof.

Hetero atom containing perfluoro compounds include F-tributyl amine, F-tripropyl amine and F-N,N-dimethylcyclohexylmethylamine; perfluoro ethers such as F-2-butyltetrahydrofuran, F-2-butylfuran, F-hydrofuran, the 1,2,2,2-tetrafluoromethyl ether of F-(2,5,8-trimethyl-3,6,9-trioxa-1-dodecanol), and other hetero compounds such as F-N-methyldecahydroquinoline, F-1-methyloctahydroquinolizine, F-octahydroquinolidine and F-N-cyclohexylpyrrolidine.

Aromatic and aliphatic compounds include F-naphthalene, F-1-methyl-naphthalene, F-n-methyl-morpholine, F-n-heptane, F-dodecane and 1,2-bis-nonyl-fluorobutylethylene. Monocyclic aliphatic compounds include F-trimethylcyclohexane, F-isopropylcyclohexane, F-tetramethylcyclohexane, F-n-butylcyclohexane, F-1-methyl-4-isopropylcyclohexane, F-p-diisopropylcyclohexane and similar compounds.

Certain of the fluorine atoms of the foregoing materials may be substituted by other halogen atoms such as bromine. Included among these compounds, are, for example, monobrominated compounds such as 1-bromopentadecafluoro-4-isopropylcyclohexane, 1-bromotridecafluorohexane, 1-bromo-pentadecafluorooctane, 1-bromo-pentadecafluoro-3-isopropylcyclopentane and perfluoro-1-bromobutylisopropyl ether, or polybrominated derivatives thereof.

Perfluorinated $C_8$ or lower materials and up to $C_{18}$ or higher materials, included partially brominated analogs thereof, as well as mixtures of various different perfluoro compounds can be used in this invention.

Those of the foregoing fluorinated compounds which are solid at ambient temperature can be dissolved in a suitable solvent or in other perfluoro compounds which are liquid at ambient temperatures, and the resulting mixture can be used to form the dispersions of the invention. "Liquid" in this specification when describing the fluoro compounds therefore means either a fluoro compound which is per se liquid at ambient temperatures or a solution of a solid fluoro compound in a fluoro compound solvent.

The more preferred perfluoro compounds for use in the invention on the basis of relative inertness (chemical and biological), good dispersability and residence time are the perfluoro $C_9$-$C_{18}$ polycyclic hydrocarbons of U.S. patent 4,105,798, and particularly F-dimethyladamantane, F-trimethylbicyclo [3.3.1] nonane, F-tricyclo [5.2.1.$0^{2,6}$] decane, F-methyltricyclo [5.2.1.$0^{2,6}$] decane, F-bicyclo [5.2.0] decane and F-methylbicyclo [5.2.0] decane, including any isomers thereof, and mixtures of such compounds, for example mixtures of F-dimethyladamantane and F-trimethylbicyclo [3.3.1] nonane, ranging from about 90/10 to 10/90 by weight.

The preferred dispersants for uniformly dispersing the perfluoro compounds in an aqueous medium are the nonionic surfactants. In some compositions and systems of the invention, particularly those cases where the dispersions are used non-systemically, such as in topical or local treatments, ionic or amphoteric surfactants may be used to disperse the perfluoro compounds. Because systemic treatments require careful attention to physiological acceptability of the compounds, such as isotonic character, ionic surfactants are less desirable, although it is possible to offset or moderate their ionic character by formulating the dispersions with electrolytes or other additives.

Suitable nonionic surfactants include aliphatic materials such as oxyethylene or oxypropylene homopolymers or block copolymers of ethylene oxide and propylene oxide comprising a hydrophobic propylene oxide section combined with one or more hydrophilic ethylene oxide sections, for example the "Pluronic" (trademark) surfactants available from BASF-Wyandotte, Inc. Less desirably, aromatic types may also be used, such as alkylphenoxypolyethoxyethanols having alkyl groups of about 7 to 18 carbon atoms and 1 to 60 or more oxyethylene units, for example: heptylphenoxypolyethoxyethanols, octylphenoxypolyethoxyethanols, methyloctylphenoxypolyethoxyethanols, nonylphenoxypolyethoxyethanols, dodecylphenoxypolyethoxyethanols, and the like; polyethoxyethanol derivatives of methylene linked alkylphenols; sulfur-containing analogs of the foregoing; ethylene oxide derivatives of long-chain carboxylic acids, such as lauric, myristic, palmitic, oleic, and the like or mixtures of acids such as are found in tall oil containing 1 to 60 oxyethylene units per molecule; and analogous ethylene oxide condensates of long-chain or branched-chain amines, such as dodecylamine, hexadecylamine, and octadecylamine, containing 1 to 60 oxyethylene groups.

Naturally occurring emulsifiers or derivatives thereof are also useful. These include the alginates,

cellulose derivatives such as methyl cellulose and carboxymethyl cellulose, water soluble gums such as gum arabic and gum tragacanth, the phospholipids (such as lecithin and yolk phospholipid as described in U.S. Patent 4,397,870-Sloviter), and the sterols.

Nonionic fluorine containing surfactants are particularly preferred. The fluorinated alkyl esters are one class of these surfactants, and are commercially available from 3M Company under the designations FC-93, FC-95, FC-128, FC-143, FC-430 and FC-431.

The more preferred nonionic, fluorine containing surfactants, from the standpoint of their exceptional ability to form dispersions which maintain a range of small particle size over substantial periods of time, of the order of 35 weeks to a year or more, even at room temperature, are the fluorinated amidoamine oxides described in U.S. patents 3,828,085 to Price et al, and 3,547,995 to Bartlett. These compounds may be generically described by the formula (1):

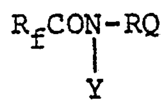
(1)

wherein $R_f$ is a perfluoroalkyl radical of 1 to about 25 carbon atoms or a polyfluoroalkoxyalkyl radical wherein the alkoxy group may contain 3 to about 40 carbon atoms of which at least a major portion thereof are perfluorinated and the alkyl group may contain 2 to about 40 carbon atoms, fluorinated or unfluorinated, Y is hydrogen or alkyl of 1 to 6 carbon atoms; R is an alkylene radical of the formula:

$-C_zH_{2z}-$

wherein z is an integer of 1 to 6; and Q is an aliphatic amine oxide radical of the formula:

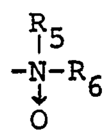

wherein $R_5$ and $R_6$ are each alkyl radicals of 1 to 6 carbon atoms or hydroxy-terminated alkyl radicals of 2 to 6 carbon atoms. In all cases the alkoxy, alkyl and alkylene groups may be straight or branched chain.

Preferred subclasses of the surfactants of the foregoing patents are those of the following formulas (2) and (3):

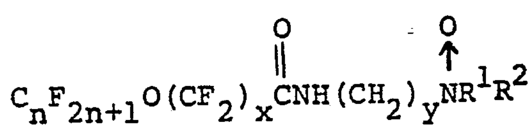
(2)

wherein n is at least 3 (preferably 3-10), x is at least 2 (preferably 2-6), y is at least 1 (preferably 2-6), and $R^1$ and $R^2$ independently are alkyl radicals containing 1-6 carbon atoms; and

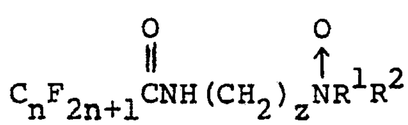
(3)

wherein n is at least 3 (preferably 3-10), z is at least 1 (preferably 2-6), and $R^1$ and $R^2$ independently are alkyl radicals containing 1-6 carbon atoms.

Specific amidoamine oxides within the scope of formula (1) are described in Examples 1-6 of U.S. Patent 3,828,085, namely:

$$CF_3(CF_2)_6\overset{O}{\overset{\|}{C}}NH(CH_2)_3\overset{O}{\overset{\uparrow}{N}}(CH_3)_2$$

$$(CF_3)_2CFO(CF_2)_3\overset{O}{\overset{\|}{C}}NH(CH_2)_3\overset{O}{\overset{\uparrow}{N}}(CH_3)_2$$

$$(CF_3)_2CFO(CF_2)_5\overset{O}{\overset{\|}{C}}NH(CH_2)_3\overset{O}{\overset{\uparrow}{N}}(CH_3)_2$$

$$(CF_3)_2CFO(CF_2)_7\overset{O}{\overset{\|}{C}}NH(CH_2)_3\overset{O}{\overset{\uparrow}{N}}(CH_3)_2$$

$$(CF_3)_2CFO(CF_2)_5\overset{O}{\overset{\|}{C}}NH(CH_2)_3\overset{O}{\overset{\uparrow}{N}}(C_2H_5)_2$$

$$(CF_3)_2CFO(CF_2)_8(CH_2)_{10}\overset{O}{\overset{\|}{C}}NH(CH_2)_3\overset{O}{\overset{\uparrow}{N}}(C_2H_5)_2$$

To provide the initial dispersion, conventional ready-made perfluoro compound dispersions may be supplied, or the dispersions may be prepared by blending the perfluoro compound and principal surfactant into water in any amounts and proportions which will provide uniform dispersions. Typical amounts are 5 to 75% (w/v) of perfluoro compound and 1.0 to 15% (w/v) of the surfactant, based on total volume of dispersion, i.e., 5 to 75 g. of perfluoro compound, and 1 to 15 g. of surfactant, per 100 ml. of total dispersion. Preferred amounts are 5-25% (w/v) of the perfluoro compound and 2-10% (w/v) of the surfactant, on the same basis. However, the invention now permits, as standard practice, the preparation of dispersion in which the perfluoro compound is initially present in the range of 25 to 60% (w/v), and the principal surfactant is initially present at 10 to 20% (w/v), preferably 5 to 10% (w/v).

The aqueous dispersions (both initially and finally) more usually comprise emulsion, preferably of the oil-in-water type but also including water-in-oil emulsions. In some cases the emulsions have a very fine particle size and appear transparent or solution-like to the unaided eye. The microemulsions which can be formulated with the dispersants of U.S. patent 3,828,085 have this characteristic and are preferred. Colloidal suspensions, while not excluded from use in this invention, are less preferred, particularly for systemic administration, because of their larger particle size range and lower stability.

The mixture of perfluoro compound, water and surfactant is dispersed by any conventional means of agitation, for example, by hand stirring, aeration, propeller agitation, turbine agitation, colloid milling, homogenizing, high-frequency or ultrasonic oscillation (sonication), including combinations of these techniques. In most instances emulsification is effective at ambient temperature. However, with some of the foregoing agitation means, excess heat may be generated during the formation of the emulsion and may be removed by known means, e.g., cooling jacket. The amount of mechanical energy input from the various agitation means can vary substantially depending on, for example, the amount of material being worked and the equipment used. Preferably, a coarse emulsion is first prepared with mild agitation, as in a Waring Blender. The emulsion is then transferred to a homogenizer for completion of the emulsification and formation of the initial dispersion.

In the second step of preparing the dispersions of the invention, the perfluoro compound phase of the dispersion is concentrated to form a first phase comprising concentrated perfluoro compound-containing particles with the complexed surfactant, and a second, aqueous phase. One method of concentration is high speed centrifugation, for example at about 10,000 to 20,000 rpm, for 0.5 to 3 hours. The selection of speed and the duration of centrifugation will depend on the type and proportion of perfluoro compound in the dispersion (the less dense or the less the amount of perfluoro compound, the greater the speed). In some batch centrifugations the result is a clear, supernatant liquid layer which rises to the top and a perfluoro

compound-containing layer which falls to the bottom of the vessel. Cross flow filtration (described below) also provides a commercially proven technique which can be adapted to continuous concentration of the perfluoro compound phase.

In the light of the present specification, a person skilled in the art can select conditions which will produce the desired extent of concentration of the perfluoro compound phase. Preferably, the concentration will be such that at least 50% by weight of the surfactant originally in the dispersion is removed into the aqueous phase. Preferably not more than about 90 to 95% by weight of the surfactant is removed. The limits of the extent of surfactant removal will depend on the characteristics of the particular dispersion involved in a given instance, but can be determined by a person skilled in the art in the light of the present specification. In any event, the degree of concentration is not so great as to break the dispersion or to adversely affect the ability of the concentrated emulsion to be redispersed in added water in the third step of the process of the invention.

In the third step of the process, the two phases are physically separated, by decanting or similar means, thus removing the concentrated perfluoro compound phase from the aqueous phase containing free surfactant.

The foregoing description of the second and third steps is a sequential concentration and separation process. These steps may also be effected simultaneously by microfiltration (also known as "ultrafiltration"). Dispersions of this invention have particle sizes in the range of from 0.05 to 10 $\mu$m (microns) and therefore microfiltration may be a practical method of simultaneously concentrating the emulsion to form the concentrated perfluoro compound phase and separating such phase from the aqueous phase. Suitable microfiltration membranes include products available from the Millipore Company and Amicon Corporation as described for example, in U.S. patents 3,615,024 and 3,856,569. In microfiltration, the dispersion is supplied to one side of a membrane and a pressure differential is applied across the membrane, so that a portion of the dispersion (the aqueous phase) passes through the membrane. The portion remaining on the supply side of the membrane is the perfluoro compound phase. Pressure or vacuum filtering or decantation can be used in conjunction with microfiltration, if desired. Pressure filtration is preferred over vacuum filtration due to a tendency to foaming during vacuum filtration.

In the last step of preparing the dispersions of the invention, the separated, concentrated perfluoro compound phase material, comprising perfluoro compound particles containing a small, residual amount of surfactant complexed therewith, is agitated in an aqueous medium (such as distilled or sterilized water) to redisperse the material. Conventional agitation or mixing means and conditions are employed. The amount of perfluoro compound in the final dispersion preferably will be 20-75% (w/v) and the amount of principal surfactant preferably will be 0.1-3.0% (w/v). The final dispersions intended for internal therapeutic use typically will have an average particle size of from 0.05 to 0.6 $\mu$m (micron), and preferably will maintain an average particle size of less than 0.3 micron (e.g., 0.1 to 0.28 microns) for long periods, of the order of 35 weeks or more, at room temperature, thus indicating good stability.

Cross-flow filtration can be used to continuously reduce the concentration of undesired surfactant in the aqueous phase of an initial dispersion and to form a final dispersion having a desired concentration of perfluoro compound material. In this method, an aqueous medium (not containing the surfactant it is desired to remove) is added intermittently or continuously to a filtration vessel charged with initial perfluoro compound dispersion. As the filtration proceeds with concentration and separation of the perfluoro compound phase of the dispersion, the aqueous medium which is added replaces or "washes out" the aqueous phase of the initial dispersion. This results in controlled dilution of the aqueous phase of the initial dispersion, such that water containing undesired surfactant is removed and the concentration of undesired surfactant in the remaining aqueous phase is substantially reduced or eliminated. Also, a desired concentration of perfluoro compound phase material is obtained.

Optionally, a non-toxic auxiliary surfactant (such as lecithin or yolk phospholipid) may be added to the aqueous medium prior to the redispersion or may be added during or after the redispersion to further promote the redispersion. From 0.1 to 5% (w/v) of such surfactant may be useful. Other agents may be added as desired during the redispersion in place of or in addition to the auxiliary surfactant, such as coupling agents and cryogenic agents. The resultant dispersion may be milky or transparent, depending on the perfluoro compound, surfactants and proportions, and the other additives.

The foregoing process for preparing dispersions according to the invention can be modified to collect, screen or classify the particles comprising the concentrated, perfluoro compound phase in order to assist in obtaining a desired particle size range in the final dispersion. Commercial centrifugation systems developed for recovery and concentration of antibiotics, protein molecules or viruses, for example, are readily adaptable to separation and/or classification of the perfluoro compound containing particles of the invention and to removal of uncomplexed surfactant. The high density of the particles as compared to the aqueous

phase of the dispersions makes such systems well suited to practice of the invention. Moreover, the separation or classification may be practiced batch-wise or continuously, depending on the type of centrifugation apparatus.

For example, the De Laval countercurrent separator comprising a cylinder spinning on its vertical axis and having apertures radially spaced-apart on a horizontal cover plate on the upper end, may receive initial dispersion through the lower end. The dispersion will rise in the cylinder over the interior wall and the particles are then classified through the apertures of the plate into a plurality of streams such that desired, fine particles can be segregated from undesired, coarse particles. Simultaneously, aqueous medium containing uncomplexed surfactant is withdrawn. Further classification can be practiced on any of the streams or any of the streams can be sent to a redispersion station for preparation of the final dispersion.

A tubular bowl centrifuge permits not only classification in the manner of the cylindrical centrifuge described above but also will permit continuous concentration of the perfluoro compound phase, separation and redispersion. In this system, the initial dispersion is admitted near the lowest point of a rotating bowl. The perfluoro compound phase then separates from the aqueous phase and divides into several zones over the interior wall of the bowl according to particle size. Perfluoro compound particles of desired sizes containing complexed surfactant may then be harvested and sent to redispersion while off-size particles may be discarded or recycled for repeat of initial dispersion. The aqueous phase containing free surfactant separated from the initial dispersion may be discarded, recovered or recycled for initial dispersion of perfluoro compound.

It is also practical by application of other known systems to avoid a total separation of the perfluoro compound phase from the aqueous phase of the initial dispersion, and to continuously form the final dispersion. One such technique is countercurrent centrifugal chromatography. In this process, the initial dispersion is continuously concentrated and classified according to desired particle size ranges, while aqueous medium containing free principal surfactant is drawn off, and aqueous medium (with or without an auxiliary surfactant) is added for the redispersion.

The foregoing and other techniques for practicing the process of the invention in a batch, continuous or semi-continuous manner are described in the technical literature such as in Handbook of Separation Techniques for Chemical Engineers, Schweitzer, P.A., Ed., McGraw-Hill Book Co., New York, 1975, specifically Section 4.5 entitled "Centrifugation".

When finally formulating the dispersions of the invention for systemic administration, it is desirable to add electrolytes and other materials to render the dispersions physiologically acceptable (such as isotonic with mammalian cells), and to adjust the pH, as necessary. A suitable pH range is 7.2-7.4. Among the additives conventionally used to render fluids physiologically acceptable are buffers such as sodium bicarbonate and mixtures such as Ringer's Solution. Other materials conventionally employed in pharmaceutical preparations and known to the skilled formulator may also be added to the dispersions. These include viscosity modifiers, stabilizers (against degradation due to freezing or contamination, for example), cryogenic preservatives, diluents and encoding agents. Among such additives may be mentioned glycerin, dimethylsulfoxide, various gelatins both natural and synthetic, and polyols such as sorbitol.

In using dispersions of the invention for drug delivery, the drug if sufficiently lipophilic may be admixed with the perfluoro compound in desired proportions. This mixture can then be used to form the initial perfluoro compound dispersion, which is then processed to the final perfluoro compound dispersion in accordance with the method of the invention. Alternatively the drug may be added in a desired amount to the concentrated perfluoro compound phase resulting from the concentration and separation steps of the invention, followed by the redispersion step to form a final dispersion containing the drug. As a third approach, the drug may be added to the final perfluoro compound dispersion in the requisite amount. The lipophilicity of the drug relative to the perfluoro compound material is an important consideration for selection of drugs for effective delivery, the more lipophilic the drug, the more successful will be the delivery, as described in the aforementioned European Patent Publication A-105584.

The invention is further described and illustrated in the following examples.

## EXAMPLE 1

An initial emulsion was prepared in a conventional manner (U.S. Patent 4,105,798) by dissolving Pluronic F-68 polyoxyethylenepolyoxypropylene copolymer surfactant (molecular weight about 8200) in distilled water to a concentration of 4.375%(w/v) and then adding to the solution to a concentration of 25%-(w/v) a purified perfluorocarbon (perfluoro compound) consisting of a liquid mixture (about 80/20 by weight) of F-1,3-dimethyladamantane and F-trimethylbicyclo [3.3.1] nonane. The resulting composition is first admixed in a Waring Blender to form a crude dispersion which is then transferred to a Mantin-Gaulin

homogenizer and admixed to form a stable, uniform, clear emulsion.

Equal amounts of the emulsion were added to the first and second tubes of a two tube laboratory centrifuge and spun for 30 minutes at 12,500 RPM and relative centrifugal force maximum g's of 20,000 to concentrate the perfluoro compound phase. Each sample separated into a top supernatant phase and a bottom gelatinous perfluoro compound phase of which, in the first tube, 28.7 g. was supernatant and 15.4 g. was gel and, in the second tube, 28.5 g. was supernatant and 15.5 g. was gel. The two phases of each tube were separated by decanting and both supernatants were found to have a density of 1.0148 indicating that of the original 25% perfluoro compound, 23.9% was in the gel and only 1.1% was in the supernatant. The gel from the second tube was shaken with 41 ml. of distilled water and was found to disperse well to a substantially transparent emulsion containing 20% (w/v) of the perfluoro compound (by GC analysis). Particle size analysis with laser spectroscopy indicated a mean average particle size of 0.17 µm (micron). The emulsion remained clear and stable after 104 days storage at 5° C. (average). At the end of that time, the average particle size was 0.24 µm (micron).

Since the bulk of the initially formed emulsion upon centrifugation and separation was a liquid material which was not used for the redispersion, most of the original surfactant was not present in the redispersed emulsion; however, a small but sufficient amount had remained with the perfluoro compound phase to enable the perfluoro compound material to redisperse when shaken with the distilled water. Accordingly, the final dispersion contained a greatly reduced amount of the surfactant as compared with the amount present in the original emulsion, but essentially all of the surfactant in the final emulsion remained complexed with the perfluoro compound particles and only a minute amount, if any, transferred into the continuous phase.

EXAMPLE 2

In another experiment, conducted essentially as described in Example 1, an initial, conventionally prepared, stable and uniform emulsion containing 25% (w/v) of the same perfluoro compound, 4.357% (w/v) of the same surfactant and 1.25% (w/v) of glycerin (as a cryogenic agent) was found to have a viscosity of 2.5 mPas (centipoise) at room temperature and a mean average particle size of 0.11 µm (micron) with less than 1% of the particles exceeding 0.3 µm (micron). The emulsion was centrifuged at 3500 RPM for 6.1 hours at 3100 relative centrifugal force maximum g's (force of gravity) whereupon the two phases separated into a supernatant top layer and a gelatinous perfluoro compound bottom layer. The gel was separated from the supernatant and portions of the gel were redispersed in amounts of distilled water sufficient to provide two final emulsions, an emulsion A containing 25% (w/v) of the perfluoro compound and 1.9% (w/v) of the surfactant, and an emulsion B containing 50% (w/v) of the perfluoro compound and 3.4% (w/v) of the surfactant

The reduced amount of surfactant in the final emulsions relative to perfluoro compound concentration indicated that the bulk of the surfactant present in the initially formed dispersion had been removed with the supernatants. The final emulsions were clear, uniform and stable. Emulsion A had a viscosity of 1.3 $mm^2/s$ (centistokes). Emulsion B had a viscosity of 3.1 $mm^2/s$ (centistokes). From a plot of viscosity versus perfluoro compound concentration of the initial and final emulsions it was determined that an emulsion prepared in accordance with the invention could contain up to about 42.7% (w/v) of the perfluoro compound without an increase in viscosity as compared with the initial emulsion. This example thus demonstrates the ability, in accordance with the invention, to substantially reduce the concentration of surfactant while providing, at an acceptable viscosity, a higher concentration of perfluoro compound than was heretofore deemed possible, without diminishing the uniformity, clarity and stability of the emulsion.

EXAMPLE 3

A series of experiments was conducted to study particle size, viscosity and stability of reconstituted emulsions prepared in accordance with the invention relative to perfluoro compound and surfactant concentrations as compared with conventionally prepared emulsions. The perfluoro compound and surfactant components were the same as in Example 2. Any differences in concentrations of the components and test conditions are shown in Table I below together with the test results wherein "PFC" means perfluoro compound. Runs 1, 4 and 5 comprise the experiment of Example 2. Run 10 is the experiment of Example 1. Runs 1-3 are controls, i.e., initial, conventionally prepared, stable emulsions which were not centrifuged as were the emulsions used to prepare the reconstituted emulsions of runs 4-10. The reconstituted emulsions of runs 4-9 were prepared as described in Example 2, i.e., by centrifuging samples of the control emulsion of run 1, separating the resulting gel and supernatant layers, and redispersing the gels in amounts of distilled water sufficient to provide the indicated perfluoro compound concentrations. The surfactant

concentrations were calculated as differences between the initially known concentrations and concentrations in the separated supernatant solutions. Initial surfactant concentrations and perfluoro compound concentrations were determined by density and chromatographic analysis for the perfluoro compounds.

The results show that stable, low viscosity emulsions can be effectively prepared in accordance with the invention to contain substantially greater concentrations of perfluoro compound and lower concentrations of surfactant than were achievable in the past. Consequently, the emulsions will have greater capacity for gas transfer but with reduced toxicity due to surfactant, and therefore, will provide substantially improved therapeutic benefits.

TABLE I

| RUN | Gel Refrigeration, Days (5°C., avg.) | Emulsion Refrigeration, Days (5°C., avg.) | Centrifuge Speed, RPM | Centrifuge Time, hrs. | Concentrations, % (w/v) PFC | Glycerin | Surfactant | Supernatant Density, g/cc | Dispersion Viscosity, (CPS) MPas | Particle Size, Vol. Avg., (micron) µm |
|---|---|---|---|---|---|---|---|---|---|---|
| Controls | | | | | | | | | | |
| 1 | - | 0 | - | - | 25 | 1.25 | 4.375 | 1.1289 | 2.5 | 0.11 |
| 2 | - | 1 | - | - | 25 | 1.25 | 4.375 | 1.1289 | 2.5 | 0.11 |
| 3 | - | 8 | - | - | 25 | 1.25 | 4.375 | 1.1289 | | 0.135 |
| 4 | 7 | 1 | 3500 | 6.1 | 25 | 0.24 | 1.9 | 1.1249 | 1.3 | 0.145 |
| 5 | 7 | 1 | 3500 | 6.1 | 50 | 0.48 | 3.4 | 1.2520 | 3.1 | 0.147 |
| 6 | 7 | 8 | 3500 | 6.1 | 25 | 0.24 | 1.9 | 1.1249 | | 0.185 |
| 7 | 7 | 8 | 3500 | 6.1 | 50 | 0.48 | 3.4 | 1.2520 | | 0.260 |
| 8 | 21 | 1* | 3500 | 6.1 | 25 | | | | | 0.195 |
| 9 | 7 | 15 | 3500 | 6.1 | 25 | 0.24 | 1.9 | 1.1249 | | 0.185 |
| 10 | 1 | 104 | 12,500** | 0.5 | 25 | | | 1.0148 | | 0.240 |

*Not refrigerated - maintained at room temperature.

**Lourdes Angle Head Tabletop centrifuge operating at 20,000 relative centrifugal force (RCF) maximum g's. All other centrifugations were with a DPR 6000 Swinging Bucket Head centrifuge, International Equipment Company, operating at 3100 relative centrifugal force maximum g's.

## Claims

1. A gas and drug transporting composition comprising a stable, uniform, aqueous dispersion of a perfluoro compound and at least one surfactant, wherein at least a major proportion of the principal

surfactant present is complexed with the perfluoro compound.

2. A composition according to claim 1, wherein at least 75% by weight of the said surfactant is complexed with the perfluoro compound.

3. A composition according to either of the preceding claims, wherein the amount of the perfluoro compound is 20-75% by weight of the dispersion and the amount of the said surfactant is 0.1-3.0% by weight of the dispersion.

4. The composition of any one of the preceding claims, wherein the perfluorocompound is a non-aromatizable perfluorinated $C_9$-$C_{18}$ polycyclic hydrocarbon containing at least two bridgehead carbon atoms linked through a bridge containing at least one carbon atom, and the said surfactant is nonionic.

5. A composition according to claim 4, wherein the perfluorocompound comprises a mixture of F-dimethyladamantane and F-trimethylbicyclo[3.3.1] nonane.

6. A composition according to any one of the preceding claims, wherein the said surfactant is a fluorinated amidoamine oxide compound or a polyoxyethylene, polyoxypropylene or copolymer thereof or a phospholipid.

7. A composition according to any one of the preceding claims, wherein in addition to said surfactant, the dispersion contains in an amount less than the amount of said surfactant, a further surfactant.

8. A process for preparing an aqueous dispersion of a perfluorocompound, which comprises:
   (a) providing an initial dispersion comprising (i) a perfluoro compound phase containing surfactant complexed with the perfluoro compound, and (ii) an aqueous phase containing free principal surfactant;
   (b) concentrating the perfluoro compound phase;
   (c) separating all or a portion of the aqueous phase from the concentrated perfluoro compound phase;
   (d) redispersing the concentrated perfluoro compound phase with an amount of an aqueous medium effective to redisperse the perfluoro compound phase and thereby to form a final dispersion.

9. The process of claim 8, wherein substantially all of the aqueous phase is separated from the perfluoro compound phase in step (c).

10. The process of either of claims 8 and 9, wherein a further surfactant is added in step (d) in an amount effective to promote redispersion of the concentrated perfluoro compound phase.

11. A process according to any one of claims 8-10, wherein the concentrating of step (b) is effected by centrifugation.

12. A process according to any one of claims 8-11, wherein in the final dispersion the amount of the surfactant that was added in step a is less than the amount in the initial dispersion.

13. A process according to any one of claims 8-12, wherein the initial dispersion is prepared by agitating a mixture of perfluro compound and a surfactant in an aqueous medium.

14. A process according to any one of claims 8-13, wherein the perfluoro compound phase separated in step (c) from the initial dispersion is characterized by a range of particle sizes, and wherein the particles are classified into desired and undesired particle size range materials, and the desired particle size rage material is redispersed in step (d) and the undesired particle size range material is optionally recycled to said mixture for preparation of said initial dispersion.

15. A process according to any one of claims 8-14, wherein the composition obtained is a product according to either of claims 5 and 6.

**Revendications**

1. Composition de transport de gaz et de médicaments comprenant une dispersion aqueuse uniforme et stable d'un composé perfluoro et au moins un agent tensio-actif, où au moins une proportion majeure de l'agent tensio-actif principal présent est complexée avec le composé perfluoro.

2. Composition selon la revendication 1, dans laquelle au moins 75% en poids dudit agent tensio-actif sont complexés avec le composé perfluoro.

3. Composition selon l'une quelconque des revendications précédentes, dans laquelle la quantité du composé perfluoro est de 20-75% en poids de la dispersion et la quantité dudit agent tensio-actif est de 0,1-3,0% en poids de la dispersion.

4. Composition de l'une quelconque des revendications précédentes, dans laquelle le composé perfluoro est un hydrocarbure polycyclique en $C_9$ à $C_{18}$ perfluoré non-aromatisable contenant au moins deux atomes de carbone têtes de pont liés par un pont contenant au moins un atome de carbone, et ledit agent tensio-actif est non-ionique.

5. Composition selon la revendication 4, dans laquelle le composé perfluoro comprend un mélange de F-diméthyladamantane et de F-trimétylbicyclo[3.3.1] nonane.

6. Composition selon l'une quelconque des revendications précédentes, dans laquelle ledit agent tensio-actif est un composé oxyde d'amidoamine fluuoré ou un polyoxyéthylène, un polyoxypropylène ou un de leurs copolymères ou un phospholipide.

7. Composition selon l'une quelconque des revendications précédentes, dans laquelle outre ledit agent tensio-actif, la dispersion contient en une quantité inférieure à la quantité dudit agent tensio-actif, un autre agent tensio-actif.

8. Procédé de préparation d'une dispersion aqueuse d'un composé perfluoro, dans lequel:
(a) on fournit une dispersion initiale comprenant (i) une phase composé perfluoro contenant un agent tensio-actif complexé avec le composé perfluoro, et (ii) une phase aqueuse contenant l'agent tensio-actif principal libre;
(b) on concentre la phase composé perfluoro;
(c) on sépare la totalité ou une partie de la phase aqueuse d'avec la phase composé perfluoro concentrée;
(d) on redisperse la phase composé perfluoro concentrée avec une quantité d'un milieu aqueux efficace pour redisperser la phase composé perfluoro et former ainsi une dispersion finale.

9. Procédé de la revendication 8, dans lequel la presque totalité de la phase aqueuse est séparée de la phase composé perfluoro dans l'étape (c).

10. Procédé de l'une ou l'autre des revendications 8 et 9, dans lequel on ajoute un autre agent tensio-actif dans l'étape (d) en une quantité efficace pour favoriser la redispersion de la phase composé perfluoro concentrée.

11. Procédé selon l'une quelconque des revendications 8-10, dans lequel la concentration de l'étape (b) s'effectue par centrifugation.

12. Procédé selon l'une quelconque des revendications 8-11, dans lequel dans la dispersion finale la quantité de l'agent tensio-actif qui est ajoutée dans l'étape (a) est inférieure à la quantité qui se trouve dans la dispersion initiale.

13. Procédé selon l'une quelconque des revendications 8-12, dans lequel on prépare la dispersion initiale en agitant un mélange de composé perfluoro et d'un agent tensio-actif dans un milieu liquide.

14. Procédé selon l'une quelconque des revendications 8-13, dans lequel la phase composé perfluoro séparée dans l'étape (c) d'avec la dispersion initiale est caractérisée par une gamme de tailles particulaires, et dans lequel les particules sont réparties en matières d'intervalles de taille particulaire

désirés et non désirés , et la matière d'intervalle de taille particulaire désiré est redispersée dans l'étape (d) et la matière d'intervalle de taille particulaire non désiré est éventuellement recyclée vers ledit mélange en vue de la préparation de ladite dispersion initiale.

**15.** Procédé selon l'une quelconque des revendications 8-14, dans lequel la composition obtenue est un produit selon l'une ou l'autre des revendications 5 et 6.

**Patentansprüche**

**1.** Gas und Medikamente transportierende Mischung, umfassend eine stabile, gleichmäßige Dispersion einer Perfluorverbindung und mindestens ein Tensid, dadurch gekennzeichnet, daß mindestens ein Großteil des hauptsächlich vorhandenen Tensids mit der Perfluorverbindung einen Komplex bildet.

**2.** Mischung nach Anspruch 1, dadurch gekennzeichnet, daß mindestens 75 Gew.-% des Tensids mit der Perfluorverbindung einen Komplex bilden.

**3.** Mischung nach einem der vorhergehenden Ansprüche, dadurch gekennzeichnet, daß die Menge der Perfluorverbindung 20 - 75 Gew.-% der Dispersion beträgt, und die Menge des Tensids 0,1 - 3,0 Gew.-% der Dispersion beträgt.

**4.** Mischung nach einem der vorhergehenden Ansprüche, dadurch gekennzeichnet, daß die Perfluorverbindung ein nichtaromatisierbarer, perfluorierter polyzyklischer $C_9$-$C_{18}$ Kohlenwasserstoff ist, der mindestens zwei Brückenkopfkohlenstoffatome enthält, die über eine Brücke verbunden sind, welche mindestens ein Kohlenstoffatom enthält, und daß das Tensid nichtionisch ist.

**5.** Mischung nach Anspruch 4, dadurch gekennzeichnet, daß die Perfluorverbindung eine Mischung aus F-Dimethyladamantan und F-Trimethylbicyclo[3.3.1]nonan enthält.

**6.** Mischung nach einem der vorhergehenden Ansprüche, dadurch gekennzeichnet, daß das Tensid eine fluorierte Amidoaminoxidverbindung ist oder ein Polyoxyethylen, Polyoxypropylen oder Copolymer derselben, oder ein Phospholipid.

**7.** Mischung nach einem der vorhergehenden Ansprüche, dadurch gekennzeichnet, daß die Dispersion außer diesem Tensid ein weiteres Tensid enthält in einer Menge, die geringer ist als die Menge dieses Tensids.

**8.** Verfahren zum Herstellen einer wäßrigen Dispersion einer Perfluorverbindung, umfassend:
(a) Herstellen einer ersten Dispersion, die (i) ein Tensid mit einer Perfluorverbindungsphase enthält, welches einen Komplex bildet mit einer Perfluorverbindung, und (ii) ein freies Haupttensid mit einer wäßrigen Phase enthält;
(b) Konzentrieren der Perfluorverbindungsphase;
(c) Abtrennen der wäßrigen Phase ganz oder teilweise von der konzentrierten Perfluorverbindungsphase;
(d) erneutes Dispergieren der konzentrierten Perfluorverbindungsphase mit einer Menge eines wäßrigen Mediums, die ausreicht, um die Perfluorverbindungsphase erneut zu dispergieren und dadurch eine endgültige Dispersion zu bilden.

**9.** Verfahren nach Anspruch 8, dadurch gekennzeichnet, daß im wesentlichen die gesamte wäßrige Phase von der Perfluorverbindungsphase in Schritt (c) abgetrennt wird.

**10.** Verfahren nach einem der Ansprüche 8 und 9, dadurch gekennzeichnet, daß ein weiteres Tensid in Schritt (d) zugegeben wird in einer Menge, die ausreicht, um das erneute Dispergieren der konzentrierten Perfluorverbindungsphase zu beschleunigen.

**11.** Verfahren nach einem der Ansprüche 8 bis 10, dadurch gekennzeichnet, daß das Konzentrieren von Schritt (b) durch Zentrifugieren erfolgt.

**12.** Verfahren nach einem der Ansprüche 8 bis 11, dadurch gekennzeichnet, daß in der endgültigen

Dispersion die Menge des in Schritt a) zugegebenen Tensids geringer ist als die Menge in der anfänglichen Dispersion.

13. Verfahren nach einem der Ansprüche 8 bis 12, dadurch gekennzeichnet, daß die anfängliche Dispersion hergestellt wird durch Rühren einer Mischung aus einer Perfluorverbindung und einem Tensid in einem wäßrigen Medium.

14. Verfahren nach einem der Ansprüche 8 bis 13, bei dem die in Schritt (c) von der anfänglichen Dispersion abgetrennte Perfluorverbindungsphase durch einen Bereich von Teilchengrößen gekennzeichnet ist, und bei dem die Teilchen nach Materialien mit Teilchengrößen in einem erwünschten und in einem unerwünschten Bereich klassifiziert sind, und das Material mit der Teilchengröße im erwünschten Bereich in Schritt (d) erneut dispergiert wird, und das Material mit der Teilchengroße im unerwünschten Bereich wahlweise zu dieser Mischung zurückgeführt wird, um die anfängliche Dispersion herzustellen.

15. Verfahren nach einem der Ansprüche 8 - 14, dadurch gekennzeichnet, daß die erhaltene Mischung ein Produkt nach einem der Ansprüche 5 und 6 ist.